# FASCICULE DE BREVET EUROPEEN

(11) **EP 1 684 664 B1**
(45) Date de publication et mention de la délivrance du brevet: **21.07.2010**
(21) Numéro de dépôt: 03786021.0
(22) Date de dépôt: 18.11.2003
(51) Int. Cl.: A61F 2/06

(54) **DISPOSITIF FORMANT ENDOPROTHESE PRESENTANT DES EXTREMITES REDUITES**
ENDOPROTHESE MIT VERJÜNGTEN ENDEN
DEVICE FORMING AN ENDOPROTHESIS HAVING TAPERED ENDS

(43) Date de publication de la demande: 02.08.2006
(73) Titulaire: Hexacath, 92500 Rueil Malmaison (FR)
(72) Inventeur: ASCHER, Gilles, F-75016 PARIS (FR)
(74) Mandataire: Portal, Gérard
(86) Numéro de dépôt international: PCT/FR2003/003417
(87) Numéro de publication internationale: WO 2005/060873

(56) Documents cités:
- EP-A- 0 688 545
- US-A- 4 580 568
- US-A1- 2002 062 145

## Description

L'invention concerne essentiellement un dispositif formant endoprothèse comprenant une extrémité proximale et une extrémité distale, comprenant un moyen facilitant le passage du dispositif au travers d'un obstacle.

### ETAT DE LA TECHNIQUE

Les dispositifs endoprothèses, dénommés dans le langage usuel "stent", permettent de traiter les rétrécissements de conduits divers de l'anatomie humaine tels qu'une artère et de les maintenir ouverts.

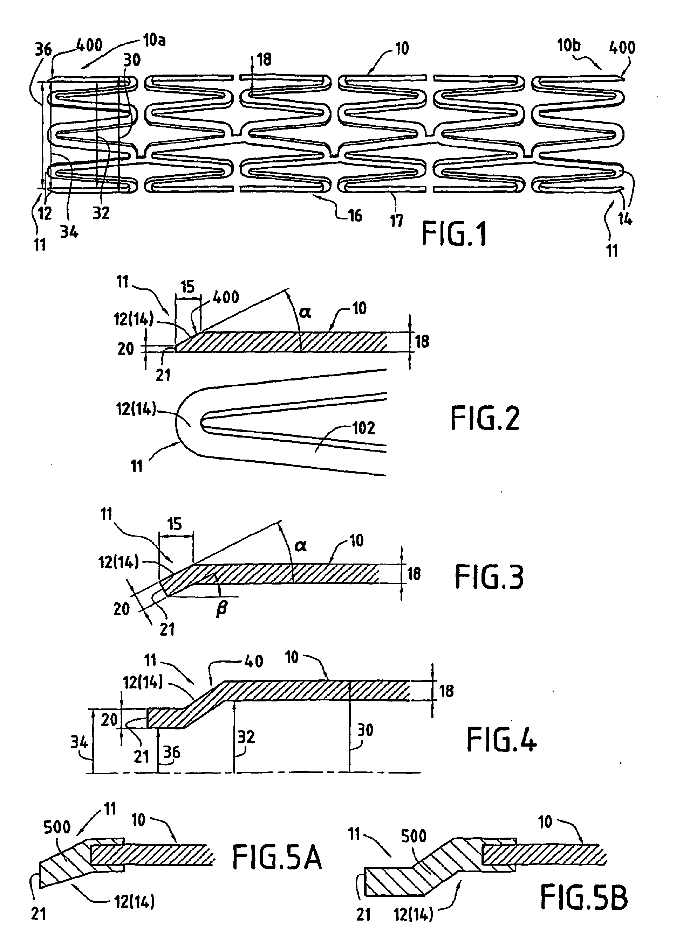

Il existe différents types de stents, qui sont décrits dans de nombreux brevets ; voir EP 0 688 545 décrivant un stent ayant un diamètre de fils relativement grand dans la partie centrale et un diamètre de fils plus faible dans les parties latérales, voir figure 5 et colonne 13 lignes 31 à 49.

Cependant pour la mise en place de ce dispositif formant endoprothèse, par exemple au cours d'une angioplastie, il est nécessaire d'utiliser un cathéter-guide ainsi qu'un guide souple.

Le cathéter-guide est placé en amont de la sténose, par exemple en amont d'une artère sténosée, le guide souple, par passage au travers de la sténose, se prolonge en aval de celle-ci.

Le dispositif formant endoprothèse avant d'être mis en position définitive est en position rétractée relativement à la sténose.

Il existe différents modes d'expansion pour ces dispositifs formant endoprothèses afin de le mettre en place, tels que notamment l'auto-expansion ou l'expansion par ballonnets.

Cependant, lorsque le conduit où doit être disposé le dispositif formant endoprothèse est trop réduit, notamment lorsque celui-ci est réduit d'un diamètre inférieur à celui du diamètre extérieur du dispositif formant endoprothèse, le manipulateur est dans l'impossibilité de disposer ce dispositif.

Le manipulateur est alors amené à retirer l'ensemble du dispositif formant endoprothèse du corps du patient.

Le manipulateur s'expose alors à une difficulté qui réside dans le fait que la partie proximale du dispositif formant endoprothèse se heurte à l'entrée du cathéter-guide, ce qui peut entraîner le dessertissage partiel, voire complet, du dispositif formant endoprothèse.

D'autre part, il se peut également que le manipulateur, lors de l'introduction du dispositif formant endoprothèse dans une lésion fortement sténosée, fasse buter le dispositif formant endoprothèse contre la partie calcifiée.

### BUTS DE L'INVENTION

La présente invention a pour but principalement de résoudre les nouveaux problèmes techniques énoncés.

L'invention a pour but de résoudre le nouveau problème technique qui consiste à trouver une solution permettant d'améliorer l'opération de retrait de l'ensemble du dispositif facilitant le placement du dispositif formant endoprothèse à l'intérieur du cathéter-guide lors d'un retrait hors du corps du patient.

La présente invention a également pour but de résoudre le nouveau problème technique consistant à faciliter le passage du dispositif formant endoprothèse au travers d'une sténose.

La présente invention a également pour but de résoudre les différents problèmes techniques énoncés en s'appliquant à toutes les formes de dispositifs formant endoprothèse ou "stents" existants, tels que notamment dispositifs formant endoprothèses tubulaires, mono-filamentaires, à maillage tressé, ou tricoté.

De plus, la présente invention a pour but de s'appliquer à tous les modes d'expansion, tels que notamment aux dispositifs formant endoprothèses auto-expansibles, ou expansibles par ballonnets.

La présente invention a également pour but d'être utilisable dans l'ensemble des domaines le nécessitant, tels que notamment la cardiologie interventionnelle et la radiologie vasculaire.

L'invention a pour but de fournir une solution aux problèmes techniques énoncés par une conception particulièrement simple, relativement peu coûteuse, utilisable à l'échelle industrielle et médicale.

### DESCRIPTION DE L'INVENTION

Ainsi, selon un premier aspect, la présente invention fournit un dispositif formant endoprothèse comprenant une extrémité proximale et une extrémité distale, caractérisé en ce qu'il comprend au moins un moyen facilitant le passage du dispositif dans un conduit, tel qu'un conduit sanguin ou un cathéter-guide, notamment au niveau d'un obstacle, par exemple une sténose ou l'extrémité d'un cathéter-guide.

L'extrémité proximale peut comprendre une pluralité de bords d'extrémité proximaux et l'extrémité distale peut comprendre une pluralité de bords d'extrémité distaux.

Selon un mode de réalisation avantageux de l'invention, le moyen facilitant le passage est disposé au moins sur certains des bords de ladite extrémité proximale ou distale, de préférence aux deux extrémités respectivement proximale et distale.

Selon un mode de réalisation avantageux, le moyen facilitant le passage comprend une section transversale réduite à au moins une extrémité, distale ou proximale.

Selon un mode de réalisation avantageux, la section transversale réduite comprend au moins une partie biseautée réalisée sur au moins un ou certains bords de l'extrémité distale ou proximale.

Selon un mode de réalisation avantageux, le dispositif formant endoprothèse, comprenant une extrémité distale et une extrémité proximale, est formé par un ou plusieurs fils métalliques enroulés en zig-zag, définissant des coudes ou bords , au moins un desdits coudes ou bords présents à l'extrémité proximale et/ou distale comprend ledit moyen facilitant le passage.

Selon un mode de réalisation avantageux, le moyen facilitant le passage est réalisé de manière mécanique dans le matériau du dispositif, par exemple par un sertissage, ou un aplatissement ou un usinage par enlèvement de copeaux.

Selon un mode de réalisation avantageux, le moyen facilitant le passage comprend un embout d'extrémité solidarisé à au moins l'un des bords d'extrémité proximale (12) ou distale (14) du dispositif.

L'extrémité proximale et/ou distale comprend de manière générale une pluralité de bords d'extrémité. Dans le cadre d'un dispositif formant endoprothèse formé par un ou plusieurs fils métalliques enroulés en zig-zag, définissant des coudes ou bords, les bords d'extrémité sont notamment formés par certains des bords présents aux extrémités du dispositif.

Avantageusement, le dispositif formant endoprothèse comprend une pluralité de moyens facilitant le passage du dispositif.

Avantageusement, le moyen facilitant le passage comprend un biseau, présentant ou non un méplat, sur au moins l'un des bords d'extrémités, et en particulier sur une extrémité d'au moins un bord précité. Le méplat est de préférence d'au moins 0,025 mm. Le méplat permet d'obtenir un bord d'extrémité moins dangereux, notamment en limitant la pointe ou épaisseur que représente ce bord d'extrémité, et en constituant une surface inclinée vers l'axe du dispositif facilitant le glissement de l'extrémité pour pénétrer soit dans le passage étroit de la sténose, soit à l'intérieur du cathéter guide en vue d'un retrait.

Avantageusement, le biseau présente un angle alpha (α) définissant l'angle du biseau, ledit angle étant compris de préférence entre 1 et 89°, et encore de préférence entre 5 et 55°.

Avantageusement, le biseau s'étend sur une longueur variant de 0,01mm à toute la longueur du dispositif. Ainsi selon une variante, au moins un fil servant à fabriquer le dispositif ou stent est biseauté.

Un mode de réalisation particulièrement avantageux est trouvé en choisissant un angle α compris entre 20 et 40° et de préférence lorsque l'angle α vaut environ 30°.

L'invention peut être réalisée en ne présentant un moyen facilitant le passage du dispositif que sur certains bords d'extrémité de l'extrémité proximale et/ou distale.

Il est évidemment entendu par les inventeurs qu'un mode de réalisation avantageux réside dans un dispositif formant endoprothèse comprenant l'ensemble des bords d'extrémité des extrémités distales ou proximales, pourvu des moyens facilitant le passage du dispositif.

L'invention peut se réaliser en effectuant l'un des modes de réalisation à l'un des bords d'extrémité, proximale ou distale, avantageusement l'invention se réalise sur l'ensemble des bords d'extrémité, proximale ou distale et, de manière encore plus avantageuse, sur l'ensemble des bords d'extrémité, proximale et distale. De ce fait, dans ce mode très avantageux de réalisation, l'extrémité proximale et l'extrémité distale présentent des bords d'extrémité comprenant chacun des moyens facilitant le passage du dispositif dans un conduit.

Le dispositif formant endoprothèse peut être réalisé à partir de fil métallique par exemple constitué d'un acier inoxydable tel que l'inox 316L, ou d'autres métaux comme par exemple le tantale, ou mélanges de métaux comme par exemple le nitinol. Ce substrat peut être revêtu d'une couche inorganique par exemple en Carbone, Carbure de Silicium, nitride d'oxyde de titane, titane, cobalt-chrome, platine, silicones, polymères. Ce substrat peut encore être revêtu d'une couche susceptible de relarguer des drogues à pouvoir antimitotique, anti-inflammatoire ou immunosuppresseur. Le diamètre du fil peut varier dans de larges limites bien connues de l'homme de l'art, généralement entre 0,05 et 0,20 mm.

### DESCRIPTION DETAILLEE DE L'INVENTION

Cette invention peut par exemple être utilisée dans le cadre du déroulement d'une angioplastie et d'une opération chirurgicale de réparation d'une sténose nécessitant la pose d'un stent ou plus généralement d'un dispositif d'endoprothése.

Notamment, le manipulateur doit, dans ce type d'opération, disposer un ensemble comprenant un cathéter-guide ; un guide généralement souple ; un cathéter porteur à ballonnet destiné à porter au moins un dispositif formant endoprothèse disposé au moins extérieurement autour du ballonnet à l'état dégonflé, dans un conduit, par exemple formé par une artère ou éventuellement une veine.

Comme il est bien connu de l'Homme de l'Art, le cathéter-guide est tout d'abord avancé en amont de la sténose. Ensuite, le guide métallique souple est introduit au travers de la sténose jusqu'en aval de celle-ci. Enfin, le cathéter porteur avec son dispositif formant endoprothèse est inséré dans le cathéter-guide en coulissant sur le guide. A ce stade, il peut arriver que le dispositif formant endoprothèse ne puisse pas pénétrer au travers du passage défini par la sténose.

La présente invention fournit un moyen tel que décrit précédemment permettant de faciliter le passage du dispositif formant endoprothèse au sein d'une sténose présentant une partie intérieure creuse. Les inventeurs entendent par « une partie intérieure creuse », le passage réduit laissé par la sténose pour permettre à un fluide, tel que le sang, de s'écouler au sein d'un conduit, tel qu'une artère ou une veine.

Cependant, si le dispositif formant endoprothèse selon l'invention ou selon l'art antérieur ne passe pas au travers de la sténose, le manipulateur se voit obligé de retirer l'ensemble {dispositif formant endoprothèse ; cathéter porteur à ballonnet} du corps du patient.

Pour cela, le manipulateur doit repositionner l'ensemble {dispositif formant endoprothèse et cathéter porteur à ballonnet} dans le cathéter-guide.

Cependant, lors de ce retrait, il se peut que le dispositif formant endoprothèse vienne buter contre l'entrée du cathéter-guide et qu'un dessertissage partiel ou complet du dispositif formant endoprothèse relativement au cathéter porteur, se produise.

La présente invention fournit des moyens précédemment décrits pour permettre la réinsertion de l'ensemble {dispositif formant endoprothèse et son cathéter porteur} dans le cathéter-guide.

D'autres buts, caractéristiques et avantages de l'invention apparaîtront clairement à la lumière de la description suivante, faite en référence aux dessins annexés, représentant plusieurs modes de réalisation actuellement préférés de l'invention, donnés simplement à titre d'illustration, et qui ne sauraient donc en aucune façon limiter la portée de l'invention.

Dans les figures :
- la figure 1 représente un premier mode de réalisation actuellement préféré d'un dispositif formant endoprothèse (10), selon la présente invention, dans lequel le moyen (11) facilite le passage du dispositif formant endoprothèse dans un conduit est un biseau (400) ;
- la figure 2 représente une vue de détail d'un bord d'extrémité, tel qu'un bord d'extrémité distal (14) et/ou un bord d'extrémité proximal (12) du dispositif de la figure 1 ;
- la figure 3 représente un autre mode de réalisation du moyen (11) de la présente invention représentant une vue de détail d'un bord d'extrémité tel qu'un bord d'extrémité distal (14) et/ou proximal (12) du dispositif ;
- la figure 4 représente un autre mode de réalisation du moyen (11) de l'invention, en représentant une vue de détail d'un bord d'extrémité, tel qu'un bord d'extrémité distal (14) et/ou proximal (12) du dispositif ;
- la figure 5 représente un mode de réalisation dans lequel le moyen (11) est un embout solidarisé (500), permettant de réduire la section d'au moins une des extrémités, proximale ou distale ;
- la figure 6 représente un mode de réalisation dans lequel le moyen (11) est un bord d'extrémité proximal (12) et/ou distal (14) ayant une souplesse suffisante pour permettre une réduction du diamètre extérieur terminal (34) permettant de franchir un obstacle en se positionnant pour délimiter un nouveau diamètre extérieur terminal (34') ;
- la figure 7 représente un dispositif formant endoprothèse (50), selon l'art antérieur, ayant des bords d'extrémité plans ;
- la figure 8 représente un dispositif formant endoprothèse (50), selon l'art antérieur, en butée contre un obstacle (100) formé ici par le cathéter guide, lors du retrait du dispositif formant endoprothèse (50) du corps 'un patient par exemple;
- la figure 9 représente un dispositif selon l'un des modes de réalisation du moyen (11) de la présente invention, notamment un dispositif formant endoprothèse (10) ayant au moins les bords d'extrémité proximaux (12) biseautés, en butée contre un obstacle, lors du retrait du dispositif, facilitant le passage du dispositif au travers de l'obstacle (100), formé ici par le cathéter guide, en permettant ainsi de pénétrer à nouveau à l'intérieur du cathéter guide;
- la figure 10 représente un dispositif (50), selon l'art antérieur en butée contre un obstacle (200) lors de la mise en place du dispositif, par exemple lors d'une intervention sur une sténose ;
- la figure 11 représente un dispositif selon l'un des modes de réalisation du moyen (11) de la présente invention, par exemple un
   dispositif formant endoprothèse (10) présentant au moins un des bords d'extrémité distaux (14) biseauté, en butée contre un obstacle (200) lors de la mise en place du dispositif, facilitant la mise en place du dispositif au travers de l'obstacle (200).
   La figure 1 représente un premier mode de réalisation actuellement préféré d'un dispositif formant endoprothèse, selon la présente invention, représenté par le numéro de référence général (10). Ce dispositif formant endoprothèse comprend une extrémité proximale (10a) définie par un ensemble de bords (12) d'extrémité proximale et une extrémité distale (10b) définie par un ensemble de bords (14) d'extrémité distale, reliées entre elles par une partie centrale (16), formant le corps du dispositif.
   Le moyen (11) est un biseau (400) dans ce mode particulier de réalisation.
   Les bords d'extrémités proximaux (12) et distaux (14) présentent un biseau, par exemple présentant un angle du biseau α ayant une valeur variant de 1 à 89°, mais de préférence étant compris entre 5 et 55°, et étant dans un mode particulier de réalisation d'environ 30°, et de préférence de 27°. Les bords d'extrémités proximaux (12) et distaux (14) présentent également un méplat (21) de longueur (20) comprise entre 0,01 mm et l'épaisseur (18) du dispositif formant endoprothèse. Ce dispositif formant endoprothèse voit avantageusement son épaisseur (18) équivalente à celle des dispositifs formant endoprothèses connus par l'homme de l'art et est généralement comprise entre 0,05 et 0,20 mm.
   Dans un mode de réalisation avantageux, l'épaisseur (18) correspond à l'épaisseur d'un ou plusieurs fils métalliques (17) enroulés en zig-zag permettant de former le dispositif formant endoprothèse.
   Le corps du dispositif ou partie centrale (16) permet de délimiter un diamètre extérieur (30) et un diamètre intérieur (32), de même qu'un diamètre extérieur terminal (34) et un diamètre intérieur terminal (36). L'invention vise dans son but général d'obtenir un diamètre (34) et/ou (36) inférieur au diamètre (30).

Cette structure permet de faciliter le passage au travers d'un obstacle présentant une partie creuse dans laquelle le dispositif formant endoprothèse doit être inséré, tel que notamment au travers d'une sténose, ou d'un cathéter-guide.

La figure 2 représente un détail du biseau (400) des bords d'extrémité proximale (12) ou distale (14) du mode de réalisation de la figure 1. Cette figure représente également une vue de dessus d'une telle extrémité, par exemple d'un dispositif formant endoprothèse aux extrémités arrondies, présentant un biseau. Cette figure permet de représenter l'épaisseur (18), ainsi que l'épaisseur (20) donnée par le méplat (21), si celui-ci est présent. De même la longueur (15) représente la longueur du biseau (400) sur la surface extérieure dans ce mode de réalisation. Le biseau peut s'étendre sur une longueur (15) variant de 0,01mm à toute la longueur du dispositif. On peut notamment identifier l'angle α représentant l'angle du biseau, cet angle pouvant varier de 1 à 89°, mais étant de préférence compris entre 5 et 55° et étant dans un mode de réalisation particulier d'environ 30° et de préférence de 27°.

La figure 3 représente un autre mode de réalisation du moyen (11), et représente notamment l'angle α défini par l'angle du biseau et représente l'angle β défini par le demi angle au sommet de intérieur du tronc de cône formé dans l'espace par l'ensemble des bords d'extrémité proximaux (12) ou distaux (14), lorsque l'ensemble de ces bords d'extrémité présente des moyens (11) facilitant le passage du dispositif.

La figure 4 représente un autre mode de réalisation du moyen (11) de la présente invention, dans lequel le dispositif formant endoprothèse (10) comprend un corps de dispositif (16) ayant un diamètre externe (30) et un diamètre interne (32). Les bords d'extrémité proximaux (12) ou distaux (14) forment dans leur ensemble un diamètre terminal extérieur (34) et un diamètre terminal intérieur (36), qui sont inférieurs aux diamètres (30) et (32) respectivement.

Dans ce mode de réalisation, l'épaisseur (18) est conservée, cependant, la partie intermédiaire (40) peut présenter une épaisseur différente. De même, le bord d'extrémité (12) ou (14) peut présenter une épaisseur (20) différente ou identique.

Dans un mode de réalisation avantageux, le bord d'extrémité (12) ou (14), dans ce mode particulier de réalisation, peut présenter une forme biseautée telle que décrite en figure 2.

Les figures 5A et 5B représentent un embout (500) biseauté solidarisé sur un dispositif formant endoprothèse, de manière à faciliter le passage du dispositif dans un conduit. Cet embout (500) est représenté dans un mode de réalisation correspondant à la figure 2. Cependant, les embouts peuvent revêtir l'ensemble des modes de réalisation concernés par l'invention et notamment les modes de réalisation prévus aux figures 3, 4 et 6.

Les figures 5A et 5B représentent de ce fait deux modes de réalisation différents d'un embout biseauté solidarisé (500), sans en limiter la forme.

Les figures 5A et 5B représentent un bord d'extrémité proximale (12) et/ou distale (14) présentant un embout solidarisé (500) à l'extrémité d'un dispositif formant endoprothèse de l'art antérieur par exemple. Cet embout solidarisé (500) est mis en place sur l'extrémité d'un dispositif formant endoprothèse présentant l'inconvénient antérieur et ne résolvant pas le problème posé et résolu par la présente invention. Cet embout solidarisé (500) peut revêtir les différentes formes prévues par l'invention dans leur généralité. Cet embout solidarisé (500) peut se présenter sous la forme d'un embout solidarisé à l'un des bords d'extrémités proximale (12) ou distale (14), ou d'un ensemble de bords d'extrémités à solidariser à l'extrémité proximale (12) ou distale (14).

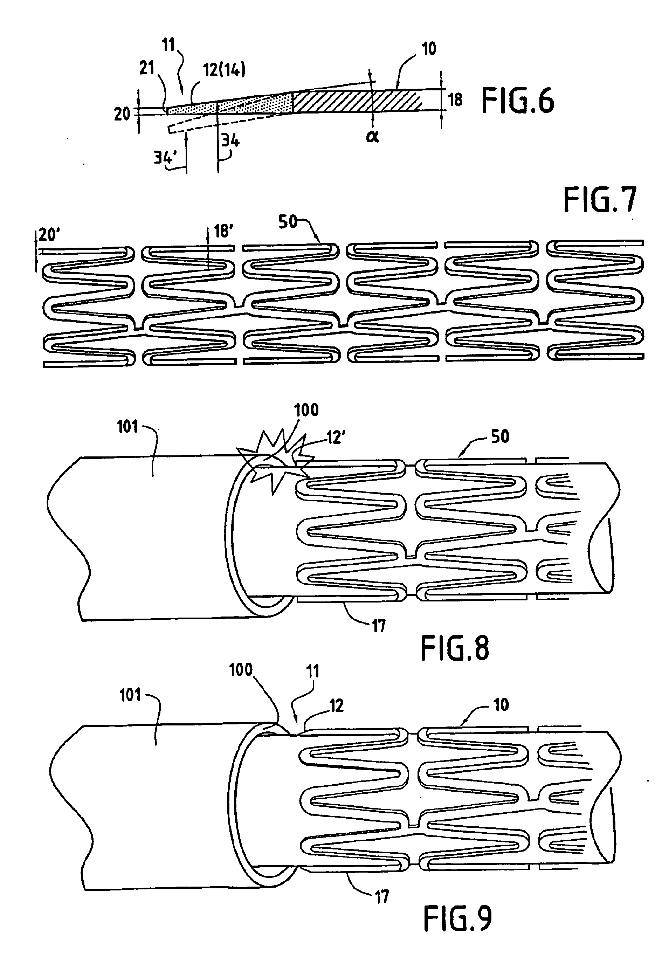

La figure 6 représente un dispositif formant endoprothèse présentant un mode de réalisation selon lequel les bords d'extrémité proximale (12) et/ou distale (14) comprennent une partie souple. Ceci permet aux dispositifs formant endoprothèse (10) de passer d'un diamètre terminal extérieur formé par l'ensemble des bords d'extrémité proximaux (12) ou distaux (14) à un nouveau diamètre terminal extérieur formé par les bords d'extrémité proximaux (12) ou distaux (14), le diamètre (34') étant inférieur au diamètre (34) et facilitant le passage du dispositif formant endoprothèse (10) au travers d'un obstacle.

La figure 7 représente un dispositif formant endoprothèse de l'art antérieur (50). Ce dispositif présente aux extrémités une forme plane, et notamment une épaisseur (20') aux extrémités identiques à l'épaisseur (18') de la partie centrale.

La figure 8 représente un dispositif formant endoprothèse de l'art antérieur (50) en butée contre un obstacle (100). Cette figure représente notamment le contact entre au moins un bord d'extrémité proximale (12') de l'art antérieur et l'obstacle (100) lors du retrait du dispositif dans un conduit (101) ; cet obstacle pouvant être le bord d'un cathéter-guide, lors du retrait du dispositif (10) dans celui-ci.

La figure 9 représente un dispositif formant endoprothèse (10) selon l'invention, par exemple selon un des modes de réalisation tels que décrits dans la figure 1 ou 2, en butée contre un obstacle (100). Cette figure permet de visualiser la manière dont est facilité le passage du dispositif formant endoprothèse (10) au travers d'un obstacle (100) grâce aux bords d'extrémité proximaux (12) pourvus de moyens (11) facilitant le passage, ici un biseau, lors d'un retrait des dispositifs formant endoprothèse dans un conduit (101) par exemple, un cathéter-guide.

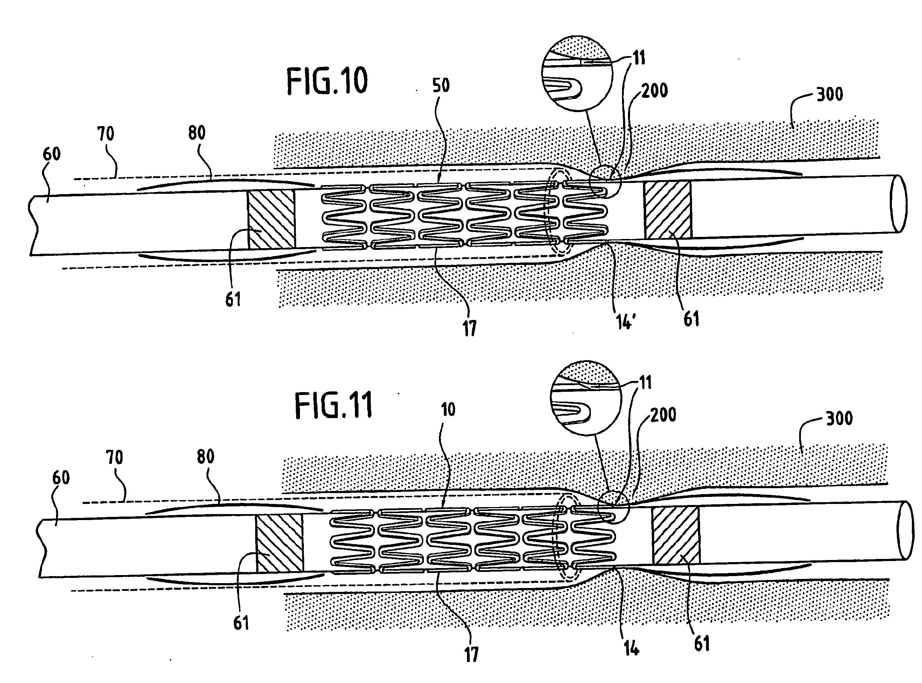

La figure 10 représente un dispositif formant endoprothèse de l'art antérieur (50) en butée contre un obstacle (200), formé par exemple par une sténose. Cette figure représente notamment le contact entre au moins un bord d'extrémité distale (14') de l'art antérieur et l'obstacle (200), qui peut être par exemple une sténose, lors de la mise en place du dispositif (50). Cette figure représente également un conduit (300) ne gênant pas le passage du dispositif formant endoprothèse, ce conduit (300) peut être par exemple une artère.

Cette figure représente également le cathéter-guide (70), le cathéter porteur à ballonnet (80), sur lequel est monté le dispositif formant endoprothèse (50) de l'art antérieur, et sur lequel est représenté des repères radiographiques (61) permettant de disposer correctement le dispositif formant endoprothèse avant son expansion. Dans cette figure, il est aisément compréhensible la difficulté que représente le passage du dispositif (50) au travers de l'obstacle (200) ;

La figure 11 représente un dispositif formant endoprothèse (10) selon invention, par exemple selon l'un des modes de réalisation tel que décrit dans la figure 1 ou 2, en butée contre un obstacle (200), formé par exemple par une sténose. Cette figure permet de visualiser la manière dont est facilité le passage du dispositif formant endoprothèse (10) au travers d'un obstacle (200), grâce aux bords d'extrémité distaux (14), par exemple lors de la mise en place du dispositif formant endoprothèse (10) au travers de cet obstacle (200).

Les références des figures sont identiques pour l'ensemble des figures 1 à 11, et le déposant n'a pas jugé nécessaire de reprendre pour la description de chaque figure l'ensemble de ces repères.

On comprend que l'invention donne lieu à divers modes de réalisation et comprend tous les moyens constituant des équivalents techniques des moyens décrits et représentés.

Les figures 1 à 11 font partie intégrante de la présente invention et donc de la présente description.

L'invention couvre aussi toute caractéristique qui apparaît nouvelle à partir de la description et des dessins eux-mêmes, par rapport à l'état de la technique et à ce titre de moyen général. On comprend également que l'on peut combiner les divers modes de réalisation et de fabrication entre eux. Il est possible de réaliser par exemple la combinaison de la restriction du diamètre de la section externe d'une extrémités distale ou proximale et d'ajuster un embout comportant également une restriction du diamètre de la section externe.

D'autres buts et caractéristiques avantageux de invention apparaîtront clairement à l'homme de l'art à partir de la description suivante faite en référence à un exemple de réalisation donnée à titre d'illustration et qui ne sauraient en aucune façon limiter la portée de l'invention.

### EXEMPLE 1

Un dispositif formant endoprothèse de l'art antérieur, par exemple, hélistent biseauté LIC7 (fabriquant : HEXACATH), est usiné par enlèvement de copeaux de manière à présenter un ensemble d'extrémités terminales proximales (12) et terminales distales (14). (Les références données par rapport aux références des figures). Ces extrémités sont usinées de manière à effectuer un biseau ayant un angle de biseau de 27°.

L'extrémité de ce biseau comprend un méplat de 0,025 mm environ.

La partie usinée s'étend sur une longueur d'environ 0,12 mm d'une extrémité.

## Revendications

1. Dispositif formant endoprothèse (10), comprenant une extrémité distale (10b), et une extrémité proximale (10a), **caractérisé en ce qu'**il comprend au moins un moyen (11) facilitant le passage du dispositif dans un conduit, tel qu'un conduit sanguin ou un cathéter-guide, notamment au niveau d'un obstacle, par exemple une sténose ou l'extrémité d'un cathéter-guide, ledit moyen (11) facilitant le passage comprenant une partie biseautée formant un biseau (400) réalisé sur au moins un ou certains bords d'extrémité proximale (12) et/ou distale (14).

2. Dispositif formant endoprothèse (10), selon la revendication 1, dans lequel l'extrémité proximale (10a) comprend une pluralité de bords d'extrémité proximaux (12) est l'extrémité distale (10b) comprend une pluralité de bords distaux (14), **caractérisé en que** ledit moyen (11) facilitant le passage est disposé au moins à un des bords d'extrémité proximale (12) ou distale (14), de préférence aux deux extrémités proximale et distale.

3. Dispositif formant endoprothèse (10), selon l'une quelconque des revendications précédentes, comprenant une extrémité distale (10b) et une extrémité proximale (10a), formé par un ou plusieurs fils métalliques (17) enroulés en zig-zag, définissant des coudes ou bords, **caractérisé en ce qu'**au moins un desdits coudes ou bords présents à l'extrémité proximale (10a) et/ou distale (10b) comprend ledit moyen (11) facilitant le passage.

4. Dispositif formant endoprothèse (10), selon l'une quelconque des revendications précédentes, **caractérisé en ce que** le moyen (11) facilitant le passage est réalisé de manière mécanique dans le matériau du dispositif, par exemple par un sertissage, ou un aplatissement, ou un usinage par enlèvement de copeaux.

5. Dispositif formant endoprothèse (10), selon l'une quelconque des revendications précédentes, **caractérisé en ce que** le moyen (11) facilitant le passage est un embout d'extrémité solidarisé (500) à au moins l'un des bords d'extrémité proximale (12) ou distale (14) du dispositif.

6. Dispositif formant endoprothèse (10), selon l'une quelconque des revendications précédentes, **caractérisé en ce qu'**il comprend une pluralité de moyens (11) facilitant le passage du dispositif.

7. Dispositif formant endoprothèse (10), selon l'une quelconque des revendications précédentes, **caractérisé en ce que** le moyen (11) facilitant le passage comprend un biseau (400) présentant un méplat (21) sur au moins l'une des extrémités proximale (10a) ou distale (10b), de préférence le méplat (21) est d'au moins 0,025 mm.

8. Dispositif formant endoprothèse (10), selon l'une quelconque des revendications précédentes, **caractérisé en ce que** au moins un des bords d'extrémité proximale (12) et/ou distale (14) présente un angle alpha (α) définissant l'angle du biseau, ledit angle étant compris de préférence entre 1 et 89°, et encore de préférence entre 5 et 55°.

9. Dispositif formant endoprothèse (10), selon la revendication 8, **caractérisé en ce que** l'angle alpha (α) est compris entre 20° et 40°, et est de préférence d'environ 30°.

10. Dispositif formant endoprothèse (10), selon l'une quelconque des revendications précédentes, **caractérisé en ce que** le moyen (11) facilitant le passage comprend un biseau s'étendant sur environ 0,12 mm sur chaque bord d'extrémité proximale (12) ou distale (14) du dispositif formant endoprothèse.

11. Dispositif formant endoprothèse (10), comprenant une extrémité distale (10b) et une extrémité proximale (10a), comprenant au moins un moyen (11) facilitant le passage du dispositif dans un conduit (101 ; 300), tel qu'un conduit sanguin, ou un cathéter-guide, notamment au niveau d'un obstacle (100 ; 200), par exemple une sténose ou l'extrémité d'un cathéter-guide, le dispositif formant endoprothèse étant formé par un ou plusieurs fils métalliques (17) enroulés en zig-zag, définissant des coudes ou bords , au moins un desdits coudes ou bords présents à l'extrémité proximale (10a) et/ou distale (10b) comprend ledit moyen (11) facilitant le passage, ledit moyen (11) facilitant le passage étant un biseau (400) réalisé sur au moins l'un desdits coudes présent à l'extrémité proximale et/ou distale, dont l'angle alpha (α) définissant l'angle du biseau est d'environ 27°, l'extrémité du biseau présente un méplat (21) d'environ 0,025 mm, le biseau étant réalisé sur une longueur (15) d'environ 0,12 mm.

## Claims

1. An endoprosthesis-forming device (10) comprising a distal end (10b) and a proximal end (10a), the device being **characterized in that** it comprises at least one passage-facilitating means (11) for facilitating passing the device into a duct, such as a blood vessel or a guide catheter, in particular in the presence of an obstacle, e.g. a stenosis or the end of a guide catheter, said passage-facilitating means (11) comprising a chamfer-forming chamfered portion (400) formed on at least one or some of the proximal end edge (12) and/or distal end edge (14).

2. An endoprosthesis-forming device (10) according to claim 1, in which the proximal end (10a) comprises a plurality of proximal end edges (12) and the distal end (10b) comprises a plurality of distal edges (14), the device being **characterized in that** said passage-facilitating means (11) is placed at at least one of the proximal end edge (12) or distal end edge (14), and preferably at both proximal and distal ends.

3. An endoprosthesis-forming device (10) according to any preceding claim, comprising a distal end (10b) and a proximal end (10a), formed by one or more zigzag-wound metal wires (17), defining bends or edges, the device being **characterized in that** at least one of said bends or edges present at the proximal end (10a) and/or distal end (10b) includes said passage-facilitating means (11).

4. An endoprosthesis-forming device (10) according to any preceding claim, **characterized in that** the passage-facilitating means (11) is made mechanically in the material of the device, e.g. by crimping, or by flattening, or by machining to remove shavings.

5. An endoprosthesis-forming device (10) according to any preceding claim, **characterized in that** the passage-facilitating means (11) is an endpiece (500) secured to at least one of the proximal end edge (12) or distal end edge (14) of the device.

6. An endoprosthesis-forming device (10) according to any preceding claim, **characterized in that** it comprises a plurality of means (11) for facilitating passing the device.

7. An endoprosthesis-forming device (10) according to any preceding claim, **characterized in that** the passage-facilitating means (11) comprises a chamfer (400) presenting a flat (21) on at least one of the proximal end (10a) and distal end (10b), preferably a flat (21) of at least 0.025 mm.

8. An endoprosthesis-forming device (10) according to any preceding claim, **characterized in that** at least one of the proximal end edge (12) and/or distal end edge (14) presents an angle (α) defining the chamfer angle, said angle preferably lying in the range 1° to 89°, and more preferably in the range 5° to 55°.

9. An endoprosthesis-forming device (10) according to claim 8, **characterized in that** the angle (α) lies in the range 20° to 40°, and is preferably about 30°.

10. An endoprosthesis-forming device (10) according to any preceding claim, **characterized in that** the passage-facilitating means (11) comprises a chamfer extending over about 0.12 mm at each proximal end edge (12) or distal end edge (14) of the endoprosthesis-forming device.

11. An endoprosthesis-forming device (10) comprising a distal end (10b) and a proximal end (10a), the device comprising at least one passage-facilitating means (11) for facilitating passing the device in a duct (101; 300) such as a blood vessel or a guide catheter, in particular in the presence of an obstacle (100; 200), e.g. a stenosis or the end of a guide catheter, the endoprosthesis-forming device being formed by one or more zigzag-wound metal wires (17) defining bends or edges, at least one of said bends or edges present at the proximal and/or distal end (10a and/or 10b) including said passage-facilitating means (11), said passage-facilitating means (11) being a chamfer (400) made in at least one of said bends present at the proximal and/or distal ends, for which the angle alpha (α) defining the chamfer angle is about 27°, the end of the chamfer presenting a flat (21) of about 0.025 mm, the chamfer being made over a length (15) of about 0.12 mm.

## Patentansprüche

1. Eine Endaprathese bildende Vorrichtung (10) mit einem distalen Ende (10b) und einem proximalen Ende (10a), **dadurch gekennzeichnet, daß** sie wenigstens ein Mittel (11) aufweist, das den Durchgang der Vorrichtung in einer Röhre, wie einem Blutkanal ader einem Führungskatheter, insbesondere im Bereich eines Hindernisses, beispielsweise einer Stenose oder dem Ende eines Führungskatheters erleichtert, wobei das den Durchgang erleichternde Mittel (11) einen abgeschrägten Teil aufweist, der eine Schräge (400) bildet, die an wenigstens einem oder einigen Rändern des proximalen Endes (12) und/oder distalen Endes (14) ausgebildet ist.

2. Eine Endoprothese bildende Vorrichtung (10) nach Anspruch 1, wobei das proximale Ende (10a) eine Vielzahl von proximalen Endrändern (12) und das distale Ende (10b) eine Vielzahl von distalen Rändern (14) aufweist, **dadurch gekennzeichnet, daß** das den Durchgang erleichternde Mittel (11) wenigstens an einem der Ränder des proximalen Endes (12) oder distalen Endes (14), vorzugsweise an beiden Enden, dem proximalen und dem distalen Ende angeordnet ist.

3. Eine Endoprothese bildende Vorrichtung (10) nach einem der vorhergehenden Ansprüche, mit einem distalen Ende (10b) und einem proximalen Ende (10a), die von einem oder mehreren zickzackförmig aufgewickelten Metalldrähten (17), welche Abwinklungen oder Ränder definieren, gebildet ist, **dadurch gekennzeichnet, daß** wenigstens eine(r) der Abwinklungen oder Ränder, die an dem proximalen Ende (10a) und/oder distalen Ende (10b) vorhanden sind, das den Durchgang erleichternde Mittel (11) aufweist.

4. Eine Endoprothese bildende Vorrichtung (10) nach einem der vorhergehenden Ansprüche, **dadurch gekennzeichnet, daß** das den Durchgang erleichternde Mittel (11) mechanisch aus dem Material der Vorrichtung gebildet ist, beispielsweise durch ein Crimpen ein Abflachen oder eine spanabhebende Bearbeitung.

5. Eine Endoprothese bildende Vorrichtung (10) nach einem der vorhergehenden Anspruche, **dadurch gekennzeichnet, daß** das den Durchgang erleichternde Mittel (11) ein endseitiges Ansatzstück (500) ist, das mit wenigstens einem der Ränder des proximalen Endes (12) oder distalen Endes (14) der Vorrichtung fest verbunden ist.

6. Eine Endoprothese bildende Vorrichtung (10) nach einem der vorhergehenden Ansprüche, **dadurch gekennzeichnet, daß** sie eine Vielzahl von den Durchgang der Vorrichtung erleichternden Mitteln (11) umfaßt.

7. Eine Endoprothese bildende Vorrichtung (10) nach einem der vorhergehenden Ansprüche, **dadurch gekennzeichnet, daß** das den Durchgang erleichternde Mittel (11) eine Schräge (400) mit einer Abflachung (21) an wenigstens einem der Enden, proximalen Ende (10a) oder distalen Ende (10b), aufweist, vorzugsweise beträgt die Abflachung (21) wenigstens 0,025 mm.

8. Eine Endoprothese bildende Vorrichtung (10) nach einem der vorhergehenden Ansprüche, **dadurch gekennzeichnet, daß** wenigstens einer der Ränder des proximalen Endes (12) und/oder distalen Endes (14) einen Winkel Alpha (α) aufweist, der den Winkel der Schräge definiert, wobei der Winkel vorzugsweise zwischen 1 und 89° und weiterhin vorzugsweise zwischen 5 und 55° beträgt.

9. Eine Endoprothese bildende Vorrichtung (10) nach Anspruch 8, **dadurch gekennzeichnet, daß** der Winkel Alpha (α) zwischen 20° und 40° und vorzugsweise etwa 30° beträgt.

10. Eine Endoprothese bildende Vorrichtung (10) nach einem der vorhergehenden Ansprüche, **dadurch gekennzeichnet, daß** das den Durchgang erleichternde Mittel (11) eine Schräge aufweist, die sich an jedem Rand des proximale Endes (12) oder distalen Endes (14) der eine Endoprothese bildenden Vorrichtung über etwa 0,12 mm erstreckt.

11. Eine Endoprothese bildende Vorrichtung (10) mit einem distalen Ende (10b) und einem proximalen Ende (10a), die wenigstens ein Mittel (11) aufweist, das den Durchgang der Vorrichtung in einer Röhre (101; 300), wie einem Blutkanal oder einem Führungskatheter, im Bereich eines Hindernisses (100; 200), beispielsweise einer Stenose oder dem Ende eines Führungskatheters erleichtert, wobei die eine Endoprothese bildende Vorrichtung von einem oder mehreren zickzackförmig aufgewickelten Metalldrähten (17), welche Abwinklungen oder Ränder, definieren, gebildet ist, wobei wenigstens eine(r) der Abwinklungen oder Ränder, die an dem proximalen Ende (10a) und/oder distalen Ende (10b) vorhanden sind, das den Durchgang erleichternde Mittel (11) aufweist, wobei das den Durchgang erleichternde Mittel (11) eine Schräge (400) ist, die an wenigstens einer der Abwinklungen ausgebildet ist, welche am proximalen und/oder distalen Ende vorhanden ist und deren Winkel Alpha (α), der den Winkel der Schräge definiert, etwa 27° beträgt, wobei das Ende der Schräge eine Abflachung (21) von etwa 0,025 mm aufweist, wobei die Schräge über eine Länge (15) von etwa 0,12 mm ausgebildet ist.
